(19) Europäisches Patentamt
European Patent Office
Office européen des brevets

(11) Veröffentlichungsnummer: **0 042 096**
**B1**

(12) # EUROPÄISCHE PATENTSCHRIFT

(45) Veröffentlichungstag der Patentschrift: **09.02.83**

(51) Int. Cl.³: **C 07 C 103/44**

(21) Anmeldenummer: **81104209.2**

(22) Anmeldetag: **02.06.81**

(54) **Verfahren zur Herstellung von N,N'-Diacetylethylendiamin.**

(30) Priorität: **12.06.80 DE 3021961**

(43) Veröffentlichungstag der Anmeldung: **23.12.81 Patentblatt 81/51**

(45) Bekanntmachung des Hinweises auf die Patenterteilung: **09.02.83 Patentblatt 83/6**

(84) Benannte Vertragsstaaten: **AT BE CH DE FR GB IT LI NL SE**

(56) Entgegenhaltungen: **DE-A-2 118 282**

(73) Patentinhaber: **BASF Aktiengesellschaft, Carl-Bosch-Strasse 38, D-6700 Ludwigshafen (DE)**

(72) Erfinder: **Koehler, Waldemar, Dr., Max-Slevogt-Strasse 28, D-6710 Frankenthal (DE)**
Erfinder: **Wostbrock, Karl-Heinz, Dr., Raiffeisenstrasse 9, D-6521 Moerstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 042 096 B1

BUNDESDRUCKEREI BERLIN

Verfahren zur Herstellung von N,N'-Diacetylethylendiamin

Die Erfindung betrifft ein Verfahren zur Herstellung von N,N'-Diacetylethylendiamin (DAED) aus Ethylendiamin und Essigsäure:

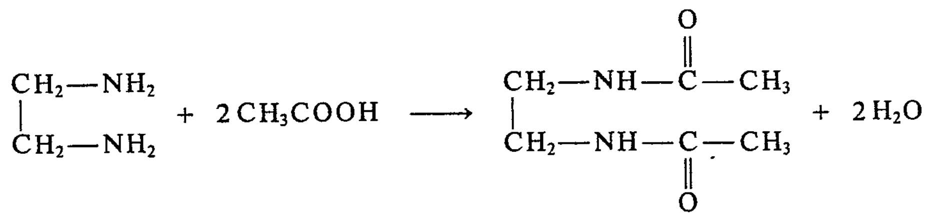

DAED kann weiter umgesetzt werden zu N,N,N',N'-Tetraacetylethylendiamin, das als Kaltbleichaktivator für perborathaltige Waschmittel dient. In den DE-PS 2 118 281 und 2 118 282 sind beide Umsetzungen beschrieben.

Die Erfindung geht aus von der Beschreibung des Verfahrens in der DE-PS 2 118 282: Demnach ist bekannt, daß DAED fortlaufend (kontinuierlich) hergestellt werden kann, indem man Ethylendiamin und Essigsäure im Gewichtsverhältnis von 1 : 2 bis 1 : 3 stetig in einem kontinuierlich durchströmten Reaktor einer Schmelze von DAED bei 140 bis 215° C zuführt und überschüssige Essigsäure sowie das bei der Umsetzung von Ethylendiamin mit Essigsäure gebildete Wasser aus dem Reaktor abdestilliert. Zur Erzielung eines möglichst vollständigen Umsatzes an Ethylendiamin und einer hohen Selektivität wird praktisch ein mehrstufiger Reaktor, z. B. eine Rührkesselkaskade mit 2 bis 4 Rührkesseln, verwendet und die Essigsäure im Überschuß, also z. B. im Molverhältnis von mehr als 2,05 angewandt.

Nachteilig ist dabei der Umstand, daß das bei der Reaktion entstehende Wasser gemeinsam mit noch nicht umgesetzter bzw. überschüssiger Essigsäure abdestilliert werden muß und die Konzentrierung der mit dem Reaktionswasser abdestillierten und somit verdünnt anfallenden Essigsäure z. B. durch Rektifikation einen erheblichen Energieeinsatz erfordert.

Die DE-OS 2 828 765 beschreibt eine mehrstufige Verfahrensführung, bei der in einer ersten Reaktionsstufe Ethylendiamin mit der stöchiometrischen Menge an wäßriger Essigsäure versetzt, anschließend Wasser aus dem Reaktionsgemisch abdestilliert und das zurückbleibende Reaktionsgemisch in einer zweiten Reaktionsstufe mit überschüssiger, konzentrierter Essigsäure weiter umgesetzt wird. Die wäßrige Essigsäure aus dieser zweiten Reaktionsstufe wird in die erste Reaktionsstufe zurückgeführt. Dieses Verfahren vermeidet zwar die Trennung der rückzuführenden Essigsäure vom Reaktionswasser, nachteilig ist jedoch die mehrstufige Verfahrensführung und der Zwang, einen erheblichen Überschuß an Essigsäure zur Versorgung der zweiten Reaktionsstufe anzuwenden.

Schließlich beschreibt die DE-AS 2 133 458 einen Weg zur Herstellung von DAED aus Ethylendiamin und Essigsäure, bei dem eine für die Weiterverarbeitung zu N,N,N',N'-Tetraacetylethylendiamin (TAED) ausreichende Produktqualität dann erhalten wird, wenn man die Entfernung des gebildeten Reaktionswassers erst einleitet, nachdem die Alkalität des Reaktionsgemisches bis zu einem Wert von 50—10% ihres anfänglichen Werts gefallen ist. Dabei ist nachteilig, daß die praktische Durchführung des Verfahrens offensichtlich an eine diskontinuierliche oder eine mehrstufige Verfahrensführung gebunden ist. Im übrigen wird beim Abdestillieren des Reaktionswassers ebenfalls verdünnte Essigsäure gewonnen.

Es besteht somit die Aufgabe, DAED aus Ethylendiamin und Essigsäure zu einer für die Weiterverarbeitung zu TAED ausreichenden Produktqualität auf einfache Weise herzustellen und dabei eine Abtrennung des anfallenden Reaktionswassers von unumgesetzter und überschüssiger Essigsäure insbesondere durch Rektifikation zu vermeiden.

Es wurde gefunden, daß man N,N'-Diacetylethylendiamin vorteilhaft erhält, wenn man Ethylendiamin und Essigsäure — vorzugsweise in einer Schmelze von N,N'-Diacetylethylendiamin, d. h. bei etwa 160 bis 230° C — umsetzt, wobei das gebildete und ggf. das mit einem Reaktionsteilnehmer zugeführte Wasser dampfförmig entfernt, z. B. über eine Kolonne abdestilliert wird, und erfindungsgemäß das Ethylendiamin mindestens teilweise mit dem Wasserdampf in Berührung bringt, bevor es dem Reaktionsgemisch zugeführt wird.

Dabei kann das molare Verhältnis von Essigsäure zur insgesamt zugeführten Ethylendiaminmenge z. B. 2,0 bis 2,5, vorzugsweise 2,0 bis 2,1 betragen.

Die praktische Durchführung des Verfahrens kann nach dem in der Zeichnung wiedergegebenen Schema gestaltet werden:

Die Gesamtmenge an Ethylendiamin (2) und Essigsäure (1) wird absatzweise oder fortlaufend einem Reaktionsbehälter (A) zugeführt, der eine Destillationskolonne (B) trägt, durch die das aus dem Reaktionsgemisch verdampfende Wasser (5) abgetrieben wird. Ein Teilstrom von Ethylendiamin (4) wird über diese Kolonne in den Reaktionsbehälter geleitet. Der Teilstrom beträgt mindestens 5%, vorzugsweise 20 bis 80% der insgesamt eingesetzten Ethylendiamin-Menge (2); er kann aber auch die Gesamtmenge an Ethylendiamin umfassen. Sofern nicht die Gesamtmenge (2) an Ethylendiamin durch

die Kolonne hindurchgeleitet wird, wird die Differenz zwischen den Strömen (2) und (4) als Strom (3) direkt in den Reaktionsbehälter geleitet. Das Mengenverhältnis richtet sich u. a. nach dem molaren Einsatzverhältnis von Essigsäure und Ethylendiamin sowie dem Wassergehalt dieser Ausgangsprodukte.

Die Kolonne enthält zwischen dem Zulauf des Ethylendiamins (4) und dem Flüssigkeitsablauf zum Reaktionsbehälter Böden, Füllkörper oder andere Einsätze bzw. Einbauten, wie sie in der Destillationstechnik als Kontakthilfen für die Einstellung eines Verdampfungsgleichgewichts zwischen dampfförmiger und flüssiger Phase gebräuchlich sind. Die Wirksamkeit derartiger Kontakthilfen innerhalb des genannten Kolonnenabschnitts soll wenigstens einem theoretischen Boden entsprechen; vorzugsweise ist eine Trennleistung von 2 bis 6 theoretischen Böden vorzusehen.

Im einfachsten Fall, den auch die Zeichnung wiedergibt, ist die Kolonne (B) direkt auf den Reaktionsbehälter (A) aufgesetzt, wobei die aus dem Reaktionsbehälter aufsteigenden Brüden zu der in die Kolonne geführten Ethylendiaminmenge im Gegenstrom geführt werden.

Es ist jedoch auch eine getrennte Anordnung von Reaktionsbehälter und Kolonne möglich, mit entsprechenden Rohrleitungen zur Zuführung der Brüden in die Kolonne sowie zum Ablauf der durch die Kolonne strömenden Flüssigkeit in den Reaktionsbehälter. Die dampfförmige und die flüssige Phase können daher sowohl im Gegenstrom als auch im Gleichstrom durch die Kolonne geführt werden. Um eine völlige Kondensation des Wasserdampfes zu verhindern, sollte das Ethylendiamin mit erhöhter Temperatur zugeführt oder der Wasserdampf ausreichend überhitzt sein.

In dem nachfolgenden Beispiel ist dies durch entsprechend hohe Temperatur der Schmelze geschehen.

Der aus der Kolonne austretende Wasserdampf kann ggf. kondensiert und abgeführt werden (5). Es kann zur Aufrechterhaltung eines ausreichend großen Flüssigkeitsstromes in der Kolonne allerdings zweckmäßig sein, einen teilweisen Rücklauf von kondensiertem Wassserdampf vorzusehen. Ebenso kann es zweckmäßig sein, oberhalb des Ethylendiamin-Zulaufs in der Kolonne einen weiteren Kolonnenabschnitt vorzusehen, um ein Übertreten geringer Mengen an Ethylendiamin in das abgehende Wasser (5) zu vermeiden.

Bei kontinuierlicher Reaktionsführung liegt die erforderliche mittlere Verweilzeit für das Reaktionsgemisch im Reaktionsbehälter je nach gewählter Reaktionstemperatur bei 10 Minuten bis 3 Stunden, vorzugsweise bei 15 Minuten bis 1 Stunde. Die zur Aufheizung der Reaktionspartner und zur Aufrechterhaltung der Reaktionstemperatur erforderliche Wärme wird dem Reaktionsgemisch auf technisch übliche Weise zugeführt, z. B. durch dampfbeheizte, im Reaktionsbehälter befindliche Heizschlangen oder durch einen außerhalb des Reaktionsbehälters installierten Wärmetauscher, über den das Reaktionsgemisch im Umlauf geleitet wird.

Für das erfindungsgemäße Verfahren können Ethylendiamin und Essigsäure in technischer Qualität verwendet werden. Die Essigsäure kann konzentriert oder als wäßrige Lösung vorliegen. Das ggf. mit Essigsäurelösung zugeführte Wasser wird auf dem gleichen Wege wie das gebildete Wasser entfernt, ohne daß ein Rektifikationsaufwand erforderlich wäre. Im Hinblick auf die Weiterverarbeitung des gebildeten Diacetylethylendiamins zu Tetraacetylethylendiamin ist es weiterhin denkbar, die aus dieser Weiterverarbeitung stammende, Essigsäureanhydrid enthaltende Essigsäure einzusetzen. In diesem Fall entspricht 1 Mol Essigsäureanhydrid 2 Molen Essigsäure und es wird eine entsprechend geringere Wasserdampfmenge freigesetzt.

Das Reaktionsgemisch wird dem Reaktionsraum (A) als Strom (6) entnommen. Seine Zusammensetzung hängt zwar von den eingestellten Reaktions- und Betriebsbedingungen ab, z. B. vom Einsatzverhältnis an Essigsäure zu Ethylendiamin und dem Aufteilungsverhältnis des Ethylendiamins in die Teilströme (3) und (4), jedoch besteht es i. a. überwiegend aus Diacetylethylendiamin, d. h. dem Zielprodukt. Daneben ist — je nach Reaktionstemperatur — ein Gehalt von 0,3 bis 3,0 Gew.-% Essigsäure und 0,5 bis 5,0 Gew.-% Wasser möglich. Nicht umgesetztes Ethylendiamin ist i. a. weder im Zielprodukt noch im Brüdenkondensat nachweisbar. Sofern also im Zielprodukt (6) und/oder im Destillat (5) Essigsäure auftritt, handelt es sich um den eingesetzten Überschuß an Essigsäure. Das erfindungsgemäße Verfahren eignet sich somit ausgezeichnet zur Einstellung des geringstmöglichen Essigsäureüberschusses bis hin zum stöchiometrischen Verhältnis der Ausgangsstoffe.

Die Vorteile des erfindungsgemäßen Verfahrens können folgendermaßen zusammengefaßt werden:

Die Umsetzung von Ethylendiamin mit Essigsäure zu Diacetylethylendiamin wird einstufig durchgeführt. Auch bei kontinuierlicher Reaktionsführung wird ein praktisch vollständiger Umsatz an Ethylendiamin erreicht. Die simultane Abtrennung von Wasser aus dem Reaktionsgemisch führt zu hoher Raum-Zeit-Ausbeute. Das abzudampfende Wasser muß nicht durch Rektifikation von der mitverdampfenden Essigsäure abgetrennt werden, diese wird vielmehr weitgehend durch eine chemische Wäsche entfernt. Es kann auch wäßrige Essigsäure eingesetzt werden, ohne daß ein Rektifikationsaufwand für eine Essigsäure/Wasser-Trennung auftritt. Das Verfahren läßt sich besonders vorteilhaft mit stöchiometrischen Mengen der Reaktionspartner ausüben.

## Beispiel

Die benutzte Anlage entspricht in der Anordnung der Zeichnung. Das Fassungsvermögen des Reaktionsbehälters (A) beträgt 40 l, die aufgesetzte Kolonne (R) ist bis zur Einlaufstelle des Ethylendiamins mit einer Füllkörperpackung von 100 cm Höhe ausgestattet. Der darüberliegende Kolonnenteil enthält ebenfalls Füllkörper mit 50 cm Packungshöhe.

Zu Beginn werden 49,2 kg/h (0,82 kMol/h) Ethylendiamin zu je gleichen Teilen als Strom (3) und Strom (4) sowie 108,6 kg/h (1,81 kMol/h) wasserfreie Essigsäure als Strom (1) in die Apparatur zugeführt. Die Temperatur im Behälter (A) wird auf 210° C gehalten. Das ablaufende Produkt (6) enthält 2 Gew.-% Wasser und 1 Gew.-% Essigsäure. Es fallen zunächst 36,0 kg/h Destillat (5) mit 78,6 Gew.-% Wasser und 21,4 Gew.-% Essigsäure an. Durch allmähliche Zurücknahme der Essigsäuremenge (1) auf 101,2 kg/h (1,96 kMol/h) entsprechend einem Molverhältnis von 2.06 läßt sich der Destillatanfall (5) z. B. auf 28,6 kg/h mit 1,0 Gew.-% Essigsäure reduzieren. Das Ethylendiamin wird praktisch vollständig umgesetzt; die erhaltene Diacetylethylendiamin-Schmelze kann unmittelbar zu N,N,N',N'-Tetraacetylethylendiamin weiterverarbeitet werden.

## Patentansprüche

1. Verfahren zur vorzugsweise fortlaufenden Umsetzung von Ethylendiamin und Essigsäure unter Bildung von N,N'-Diacetylethylendiamin, wobei das gebildete Wasser aus dem Reaktionsgemisch dampfförmig entfernt wird, dadurch gekennzeichnet, daß man den Wasserdampf mit mindestens einer Teilmenge des zugeführten Ethylendiamins wäscht.

2. Verfahren nach Anspruch 1, dadurch gekennzeichnet, daß man das Ethylendiamin dem Reaktionsgemisch mindestens teilweise im Gegenstrom zum abdestillierenden Wasserdampf zuführt.

3. Verfahren nach Anspruch 1, dadurch gekennzeichnet, daß man als Reaktionsgemisch eine Schmelze von Diacetylethylendiamin vorgibt.

4. Verfahren nach Anspruch 1, dadurch gekennzeichnet, daß man Essigsäure im stöchiometrischen Verhältnis oder im Molverhältnis unter 2.1 anwendet.

## Claims

1. Process for reacting, preferably continuously, ethylenediamine and acetic acid to form N,N'-diacetylethylenediamine, the water formed being removed from the reaction mixture in vaporous form, wherein the steam is washed with at least some of the ethylenediamine fed in.

2. Process according to claim 1, wherein at least some of the ethylenediamine is fed to the reaction mixture in countercurrent with the steam distilled off.

3. Process according to claim 1, wherein a melt of diacetylethylenediamine is taken as the reaction mixture.

4. Process according to claim 1, wherein the acetic acid is used in the stoichiometric ratio or in a molar ratio of less than 2.1.

## Revendications

1. Procédé pour la mise en réaction, de préférence continue, d'éthylène-diamine et d'acide acétique avec formation de N,N'-diacétyléthylène-diamine, l'eau formée étant éliminée du mélange réactionnel sous forme de vapeur, caractérisé en ce qu'on lave la vapeur d'eau avec au moins une quantité partielle de l'éthylène-diamine introduite.

2. Procédé selon la revendication 1, caractérisé en ce qu'on introduit l'éthylène-diamine dans le mélange réactionnel, au moins en partie à contre-courant par rapport à la vapeur d'eau qui s'élimine par distillation.

3. Procédé selon la revendication 1, caractérisé en ce qu'on introduit au préalable, en tant que mélange réactionnel, une fusion de diacétyléthylène-diamine.

4. Procédé selon la revendication 1, caractérisé en ce qu'on utilise l'acide acétique en proportion stoechiométrique ou dans un rapport molaire inférieur à 2,1.

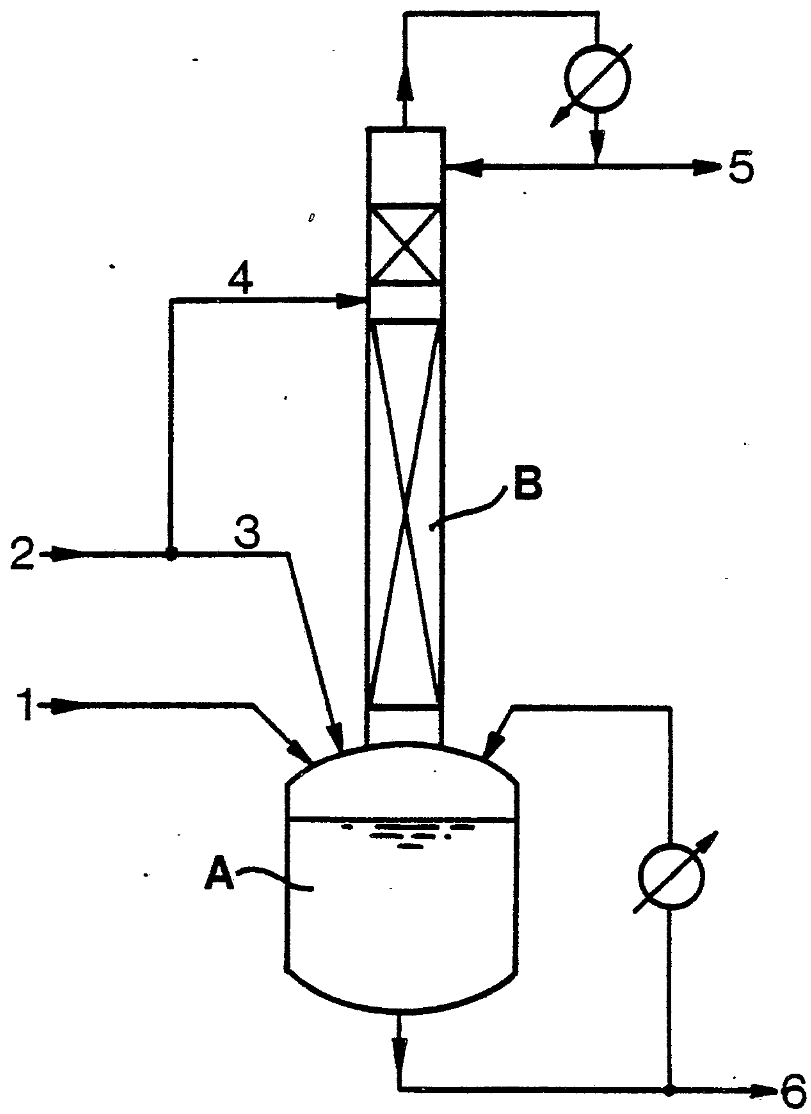
5
4
B
2
3
1
A
6